# EUROPEAN PATENT APPLICATION

(11) **EP 2 684 600 A1**
(43) Date of publication of application: **15.01.2014**
(21) Application number: 12175812.2
(22) Date of filing: 10.07.2012
(51) Int. Cl.: B01J 13/20, A01N 25/28, A23L 1/22, A23P 1/04, A61K 9/50, C09B 67/00, C11D 3/50

(54) **Core-shell capsules and methods for encapsulation of reactive ingredients by diffusional exchange through spherical capsule membranes**

(71) Applicant: Laboratoires Meiners Sarl, 2013 Colombier NE (CH)
(72) Inventor: Meiners, Jean Antoine, 2036 Cormondrèche (CH); Zegels, Alexandre, 2502 Biel (CH); Zongo, Mathieu, 1018 Lausanne (CH)
(74) Representative: Schneiter, Sorin

(57) **Abstract**

The present invention relates to a method for encapsulation and to capsules that can be obtained by the method. The method is characterized by the step of counter-current, diffusional exchange across the membrane of a capsule-shell. During the exchange, a primary core is replaced by a liquid that may comprise an active agent. The method of the invention may also comprise a step of further processing said capsules, in particular in order to adjust or modify the release characteristics and to control the release of the active agent inside the capsules.

## Description

### Technical Field

The present invention pertains to the field of encapsulation. In particular, the invention relates to a method of encapsulation, a method for obtaining capsules, capsules obtainable by the methods and core-shell capsules.

### Prior Art and the Problem Underlying the Invention

The present invention pertains to the field of encapsulation, in particular to the production of capsules, in particular microcapsules. Generally, capsules are defined according to the diameter of the preparation, as micro capsules when smaller than 1 millimetre, as nano capsules below one micrometer. Morphology of capsules depends on the process of producing them, some processes yielding spherical capsules, other processes elliptical or still other, for example irregular particulate forms.

Encapsulation is a technology that allows the chemically or biologically active substance to be confined and protected against immediate reaction with the substances in its environment. The reactive compound remains chemically identical, by physical separation from the other compounds in the system. Its release is controlled or delayed. One way of separating the chemically active core compound from its environment is to provide capsules having a core-shell configuration. The shell function is achieved by a membrane, which in many cases is expected to be very tight, or in other applications requires a gradual diffusion. The direction of the flow is designed to be in one single direction, in the majority of the cases, flowing away from the capsule core.

In most known processes, the particle size is the result from droplet separation and consequently requires a certain amount of energy to achieve the desired separation of a stream of liquid core material.

An objective of the present invention is to provide a unique platform technology, allowing the making of very small capsules as well as rather large spheres, with only minimal changes to the orifice producing the droplets. It is thus an objective of the invention, to provide a method of producing core-shell capsules that can be used for producing capsules over a relatively wide size range, capsules that can have a diameter in the range of 1 µm to 1 cm, or even larger. The variability with respect to capsule size implies that the membrane thickness differs and needs to be controllable and adjustable to the requirements of the final application.

Furthermore many core-shell capsules are not entirely proof to liquid and volatile ingredients, such as flavours or fragrances. It is thus an objective to provide capsules with a core that comprises a liquid ingredient, and which is stable for prolonged time of storage, even under dry and warm conditions.

The invention addresses further the problem of providing core-shell capsules made of materials that do not cause any concern of toxicity, in dependence of the application for which it is used.

It is also an objective to provide capsules that can conveniently be prepared from different starting materials.

Another objective of the invention is to provide capsules that have a comparatively rigid shell, which is susceptible of being ruptured upon exposure to pressure, so as to release its content at a predetermined moment.

The present invention addresses the problems depicted above.

### Summary of the Invention

Remarkably, the present inventors have provided a new method of producing core-shell capsules, which is based on diffusional exchange through the membrane of core-shell capsules. Surprisingly, the present inventors have overcome by using a multiple step process the limitations of prior art methods, which virtually all are limited to small range of capsule sizes, in general of small capsule sizes.

In an aspect, the present invention provides a method of encapsulation and/or a method of producing core-shell capsules, the method comprising the steps of: producing a shell of core-shell capsules; and, exposing said shell to a medium comprising an active agent and allowing for diffusion and/or osmosis of the active agent through said shell.

In an aspect, the present invention provides a method of encapsulation and/or a method of producing core-shell capsules, the method comprising the steps of: producing precursor core-shell capsules comprising a carrier core; exposing said precursor core-shell capsules to conditions that result in the diffusion and/or osmosis of at least part of the carrier core through the shell to the outside of the shell.

In an aspect, the present invention provides a method of encapsulation and/or a method of producing core-shell capsules, the method comprising the steps of: producing precursor core-shell capsules comprising a carrier core and an active agent; exposing said precursor core-shell capsules to conditions that result in the diffusion and/or osmosis of at least part of the carrier core through the shell to the outside of the shell, while said active agent remains substantially in the inside of said capsules.

In an aspect, the present invention provides a method of encapsulation and/or a method of producing core-shell capsules, the method comprising the steps of:
- preparing carrier beads from a carrier material,
- encapsulating said carrier beads with an appropriate film forming and/or polymeric material so as to obtain carrier core-shell capsules;
- exposing said carrier core-shell capsules to a liquid and/or solubilizing medium, wherein said carrier material is soluble or dispersible in said medium;
- removing said capsules obtained in the preceding step from said medium, so as to obtain core-shell capsules comprising an active agent;
wherein said carrier material and/or said medium comprises an active agent.

In an aspect, the present invention provides a method of preparing core-shell capsules with a core comprising an active agent, the method comprising:
- preparing a solid carrier core from a carrier material that is dissolvable in a liquid and/or solubilizing medium;
- encapsulating said solid carrier core so as to obtain carrier core-shell capsules;
- exposing said carrier core-shell capsules so said medium for a duration sufficient to at least partially replace said carrier core of said core-shell capsules by said medium, thereby obtaining core-shell capsules with a core comprising said medium;
- removing the core-shell capsules with a core from the medium;
wherein an active agent is mixed with said carrier material before and/or when forming said solid carrier core, and/or wherein an active agent is contained in said medium.

In an aspect, the present invention provides a method of encapsulation by counter current flow diffusion, the method comprising the steps of:
- preparing substantially spherical, self-sustained carrier beads from a carrier material, said carrier beads having a diameter in the range of 1 µm to 1 cm;
- encapsulate said self-sustained carrier beads with an appropriate film-forming material to form a semi-permeable shell in order to obtain precursor core shell capsules;
- exposing said precursor core-shell capsules to a solubilizing medium containing an active agent, wherein said carrier material is soluble and/or dispersible in said medium, so as to favour diffusion of the dissolved and/or dispersed carrier material out of said precursor core-shell capsules and/or diffusion of said active agent into the precursor core-shell capsules, thereby obtaining core-shell capsules comprising an active agent.

In an aspect, the invention provides capsules obtainable by any one of the methods of the invention.

In an aspect, the invention provides a core-shell capsule comprising a core comprising an active agent, wherein at least part of said core and/or of said active agent has properties of or comprises a liquid at 65°C.

In an aspect, the invention provides a core-shell capsule comprising a core comprising an active agent, wherein at least part of said core and/or said active agent is a liquid at 65°C.

In an aspect, the invention provides a capsule comprising a core at least part of which is liquid, comprises a liquid or has properties of a liquid at 45°C, preferably at 35°C and even more preferably at 25°C, and a shell, and comprising an active agent, said shell comprising cellulose acetate and/or a derivative thereof.

In an aspect, the invention provides a consumer end product comprising the capsules of the present invention.

Further aspects and preferred embodiments of the invention are defined herein below and in the appended claims. Further features and advantages of the invention will become apparent to the skilled person from the description of the preferred embodiments given below.

### Detailed Description of the Preferred Embodiments

The present invention provides methods of producing core-shell capsules, capsules that can be obtained by the method and core-shell capsules as defined herein.

The methods of the invention preferably comprise a step of preparing carrier cores or carrier beads from a carrier material. For the purpose of this specification, the expressions "carrier bead", "carrier core" and "primary core", "primary beads", "primary compound", "precursor beads" and precursor "cores" are interchangeable and may be used for describing the same principle. The expression "core" expresses the fact that said carrier beads and/or cores will form the core of intermediate or precursor core-shell capsules. The expression "primary" expresses that said carrying structure is transient and will generally be replaced by the active agent or a medium as specified elsewhere in this specification. Preferably, said carrier beads are self-sustained. The feature of being self-sustained refers to the fact that said beads are sufficiently compact so as to function as a framework, allowing for the formation of a shell around them in a further step. Said carrier beads may thus actually be liquid, for example in the form of liquid beads that are suspended in a medium, so that said shell can be formed around said carrier cores. According to a preferred embodiment, however, said beads are in a solid state, for example in the form of a crystal or a non-crystalline solid state, for example in a glassy state. Said feature of being self-sustained, for example solid, refers to the status when being subjected to the step of shell-formation as being described elsewhere in this specification.

One of the innovative aspects in the in the method of the present invention is the making of a primary, self-contained structure (core, beads), which serves as a scaffold to create a micro spherical membrane around the primary core, as will be described further below.

In a still further, subsequent step said beads will preferably be exposed to conditions in which the beads are preferably substantially disintegrated, for example solubilized and/or suspended.

According to an embodiment, said carrier beads are substantially spherical.

According to an embodiment, said carrier beads have an average diameter of 1 µm to 3 cm, preferably 1 µm to 1 cm, preferably 100 µm to 9.5 mm, more preferably 200 µm to 9.5 mm, still more preferably 400 µm to 9 mm, even more preferably 600 µm to 9 mm, and most preferably 800 µm to 8.5 mm.

According to a preferred embodiment, said carrier beads have a mean diameter of 1 to 9 mm, 1.5 to 8.5 mm preferably 2 to 8 mm, more preferably 2.5 to 7.5 mm, most preferably 3 to 7 mm, and finally 3.5 to 6.5 mm.

The carrier beads may be prepared from any suitable material. Preferred characteristics in terms of melting point, solubility and diffusion characteristics of the material that is used for preparing the carrier beads are disclosed elsewhere in this specification. According to an embodiment, said carrier material of said carrier beads is selected from PEG, water soluble saccharides, in particular water soluble mono-, di-, oligo-, and polysaccharides, lipids, fats, waxes, for example.

According to an embodiment, said carrier material may have a melting point that is ≥ 5°C, preferably ≥ 10°, more preferably ≥ 15°C, even more preferably ≥ 20°C, most preferably ≥ 25°C, in particular ≥ 30°C, ≥ 35°C, ≥ 40°C, ≥ 45°C, ≥ 50°C and ≥ 60°C.

While there exist many ways for producing carrier beads that may be used in the methods of this invention, the solidification of droplets of liquefied carrier material is cited as an example for the purpose of illustration. Accordingly, in an embodiment, said carrier beads are obtained by providing liquid carrier material (for example, liquefied carrier material) and adding droplets of liquid carrier material into a solidification medium, thereby obtaining said carrier beads by solidification of said droplets. For example, said droplets may be obtained by pumping said liquid carrier material through a nozzle into said solidification medium and/or by dropping them into the solidification medium. Preferably, said solidification medium is a liquid. Preferably, said solidification medium has a temperature that is below the melting point of said carrier material when said droplets of liquid carrier material are added.

According to an embodiment, said carrier material has a melting point that is higher than the melting point of said preferably liquid solidification medium. Therefore, when being contacted with the liquid solidification medium at a temperature that is below the melting point of said carrier material, said liquefied carrier material solidifies and forms said self-sustained carrier beads.

According to an embodiment, an active agent is added to said carrier material in said carrier beads. For example, said active agent is admixed with said carrier material before and/or when forming said carrier beads. According to an embodiment, said active agent is added to liquid carrier material. According to this embodiment, the carrier beads comprise a mixture of carrier material and active agent. In this embodiment, said carrier bead may comprise, for example, 50 to 99 wt.%, preferably 60 to 98 wt.%, more preferably 70 to 97% and most preferably 80-96 wt.% of carrier material and 1 to 50 wt.%, preferably 2 to 40 wt.wt.%, more preferably 3 to 30 wt.% and most preferably 4 to 20 wt.%, respectively, of active agent.

According to an embodiment, said active agent is selected from any chemically active or bioactive compounds. Preferably, said active agent is or comprises substances selected from the group consisting of flavours, fragrances, sweeteners, pharmaceuticals, nutraceuticals, vitamins, minerals, salts, plant extracts, enzymes, peptides, microorganisms, pigments, oxidizing agents, surfactants, pesticides, and mixtures comprising two or more of the aforementioned.

According to another embodiment, said carrier beads are substantially free of said active agent, and said active agent is added at a later moment, as disclosed elsewhere in this specification.

According to an embodiment, said carrier beads, comprising or not said active agent, may be washed with appropriate solvents, such as acetone, and/or with a blend of solvents, for example. This washing step is conducted, for example, before the subsequent encapsulation step.

In an embodiment, the methods of the invention may comprise any one or a combination of two or more of the steps selected from: producing a shell of core-shell capsules; encapsulating said carrier beads with an appropriate film forming and/or polymeric material so as to obtain carrier core-shell capsules; encapsulating said solid carrier core so as to obtain carrier core-shell capsules; and encapsulate said self-sustained carrier beads with an appropriate film-forming medium to form a semi-permeable shell in order to obtain precursor core shell capsules.

Preferably, said carrier beads are encapsulated with an appropriate material that is capable of forming a shell. In this manner, "carrier core-shell capsules", also interchangeably referred to as "precursor core shell capsules" are preferably obtained. The core of said carrier or precursor core-shell capsules thus preferably comprises, consists essentially of and/or consists of the carrier material, optionally admixed with the active agent, in case the active agent was added in the carrier beads.

Said film-forming and/or polymeric material for forming said shell may be selected, according to an embodiment, from materials comprising one or more selected from polysaccharides, polysaccharide-based materials, protein-based materials, polyvinyl alcohol, polyvinyl acetate, polylactic-co-glycolic acid, polylactic acid, and combinations comprising two or more of the aforementioned.

Examples of proteins and protein-based materials include milk proteins, such as whey protein and/or casein protein, cereal proteins, such as wheat protein and zein protein from corn, and gelatin.

Examples of polysaccharides and polysaccharide-based materials include cellulose acetate, cellulose phthalate, hydroxypropylcellulose, ethyl cellulose, cellulose derivatives in general, including other cellulose derivatives than those specified herein, chitosan, chitin, Arabic gum, alginate, pectin, pullulan, maltodextrin, cyclodextrin (e.g. cylcodextrin α, β and/or γ), starch, modified starch, and combinations comprising two and more of the aforementioned.

According to a preferred embodiment, said film-forming and/or polymeric material is or comprises cellulose and/or a derivative of cellulose, such as cellulose acetate, or a derivative of cellulose acetate, such cellulose acetate phthalate, for example. Cellulose and cellulose acetate derivatives may be advantageous in certain situations as they generally have a weak solubility in many common solvents such as pure water. They are, however, soluble in di- or triacetin and acetic acid solutions. Cellulose acetate, for example, is authorized as an ingredient for some consumer products.

As will become apparent further below, the material for preparing the shell is preferably carefully selected with respect to the membrane characteristics of the shell obtained with the material. As described below, the shell material has to allow the diffusion from the core into the solubilizing medium and, once the shell being at least partially, but preferably substantially "emptied" from its primary core, the core medium will be replaced totally or partially by the flow of the solubilizing medium and/or active compound into the micro-spherical membrane. Besides the material from which it is prepared and the encapsulating technique used, also the thickness of the shell may be adjusted in order to optimize diffusion characteristics and/or permeability of the shell.

Having the above in mind, said shell may be prepared in any suitable manner. According to an embodiment, said step of encapsulating said carrier beads comprises the step of applying said film-forming and/or polymeric material onto the solid carrier beads. According to a preferred embodiment, the step of encapsulating said carrier beads with an appropriate film forming and/or polymeric material comprises the step of coating said carrier core. For example, the coating may be performed by any one selected from the group consisting of a fluidized bed, a drum coater and a Lödige coater. If a coating technique is used for preparing said shell and/or encapsulating said core beads, said coating may be a first coating, and any one of the methods of the invention may comprise a step of applying a second coating, for example in order to change (in particular reduce) the permeability or diffusion characteristics of the shell, as is specified elsewhere in this specification.

Diffusion characteristics and/or permeability of the shell are closely related to the characteristics of pore size, pore morphology and pore distribution of the micro spherical membranes. As mentioned above, also membrane thickness may play role in the flow into and/or out of the capsules. These parameters should be kept in mind and/or should be determined or tested, in order to provide a shell that can be used for the purpose of the present invention.

The membrane or shell thickness is preferably adjusted so as to achieve the permeability and/or diffusion characteristics as desired with respect to the invention, in particular as required for the step of exposure to the solubilizing medium, where the carrier core should move to the outside of the capsule. The permeability generally depends on the thickness of the shell, and more specifically decreases with increasing thickness. The skilled person will preferably adjust the thickness of the shell so as to optimize diffusion characteristics, preferably also with regard to the material (active agent) to be encapsulated.

In accordance with the invention, the weight ratio between said carrier core and said shell of said carrier core shell capsules is in the range of 1-40 wt.% shell and 60-99 wt.% core material, preferably 2-30 wt.% shell and 70-98 wt.% core material, more preferably 3-25 wt.% shell and 75-97 wt.% core material, even more preferably 5-20 wt.% shell and 80-95 wt.% core material, and most preferably 8-15 wt.% shell and 85-92 wt.% core material, for example about 10 wt.% shell material. These weight ratios may also apply to the final capsules comprising an active agent as disclosed further below, possibly after a second coating step. In this case, the weight of the carrier core is simply replaced by the mass of the core comprising the active agent.

According to an embodiment, the shell of the carrier core-shell capsules and/or precursor core shell capsules comprises pores, optionally after further processing of the capsules obtained after encapsulating said carrier beads.

According to an embodiment, the shell of the carrier core-shell capsules and/or precursor core shell capsules is semi-permeable, optionally after further processing of the capsules obtained after encapsulating said carrier beads.

According to an embodiment, the shell of the carrier core-shell capsules and/or precursor core shell capsules has or is further processed to have membrane characteristics allowing for diffusion and/or osmosis of said solubilizing medium and/or of a liquefied and/or dissolved carrier material through said shell.

In case a particular processing step is required in order to render the shell permeable and/or porous as specified above, such a step may comprise a physical and/or a chemical treatment that brings about said permeability. For example, the carrier core-shell capsules may be exposed to a solution that is corrosive to the shell, resulting in pore formation or increase of permeability after a given time of exposure, for example. For example, a solution having a particular pH may be used to provide the necessary diffusion/permeability characteristics of the shell. Generally, physical and/or a chemical treatment that may be used include the exposure to pressure, to a change of temperature (increased and/or lowered temperatures), a change of pH, and/or combinations of two or more of these, for example.

As will be specified further below, the methods of invention may comprise a further, e.g. later step of chemically and/or physically treating the capsules to affect the diffusion and/or permeability properties of the capsules, but with the opposed goal, in particular, in order to decrease diffusion through the shell and/or permeability. In case there are two steps of physical and/or chemical treatment to modify the membrane permeability properties, the step for increasing diffusion/permeability may be a first step of chemically and/or physically treating or processing said shell, and said later step of decreasing said membrane diffusion/permeability properties may be a second, different step of chemically and/or physically treating or processing said shell.

Preferably, at this stage, optionally after said further processing step, the membrane characteristics of the shell of said carrier core shell capsules are such that said shell is permeable to at least the carrier material, optionally after solubilizing or suspending the carrier material. Preferably, the membrane characteristics of the shell at this stage are such that the shell is permeable to an active agent, which is to be encapsulated or which may be present in the capsules at this stage already.

Preferably, the membrane of said carrier core shell capsules has or is processed to have properties that optimize and/or favor the diffusion of the active agent into the capsules and/or the diffusion of a solubilized (for example melted or dissolved) and/or suspended carrier material from the inside to the outside of the capsules, in particular under conditions as specified below.

According to an embodiment, the methods of the invention may comprise the step of exposing said precursor core-shell capsules to conditions that result in the diffusion and/or osmosis of at least part of the carrier core through the shell to the outside of the shell. For example, these conditions may be provided by a medium.

The invention takes into consideration that optimal pore characteristics for diffusion out of the microcapsule may be different from the characteristics favouring the diffusion into the microcapsule.

According to an embodiment, the methods of the invention may comprise the step of exposing said carrier core-shell capsules to a solubilizing medium, preferably a liquid medium.

Preferably, said carrier material is soluble or dispersible in said medium, whether the medium is liquid or gaseous, for example.

According to an embodiment, said carrier material of said carrier beads, said active agent and said (preferably liquid) solubilizing medium are selected so that said carrier material and said active agent are both soluble or dispersible in said medium, optionally following chemical and/or physical treatment of said medium. For example, the solubilizing medium may be provided at a temperature that is above the melting point of said carrier material. In this manner, the carrier material is exposed to said temperature when said carrier core-shell capsules are exposed to said medium. Preferably, said medium is liquid at said temperature.

According to an embodiment, said active agent and/or said carrier material are soluble and/or dispersible in liquid water, optionally following heating said water to a temperature of up to 100°C, preferably up to 90°C, more preferably up to 70°C, most preferably up to 60°C.

According to an embodiment, said solubilizing medium is liquid at a temperature of 70°C, 60°C, 50°C, 40°C, 30°C, preferably 25°C, more preferably 20°, 15°C, even more preferably 10°C, 5°C, 0°C, and most preferably at 0°C or possibly even at -5°C, for example.

According to an embodiment, said medium is a liquid medium selected from the group consisting of an aqueous solution, an alcohol-containing solution, a hydrophobic solvent, oils, and mixtures comprising one or more of the aforementioned.

An exemplary way of exposing said carrier core-shell capsules so said a solubilizing medium comprises placing the carrier core-shell capsules inside a bath comprising said liquid medium. The carrier core shell capsules may simply be transferred, for example dropped into said liquid medium.

Preferably, during said step of exposing said carrier-core shell capsules to a liquid medium, a substantial part of said carrier material is liquefied, for example dissolved, melted and/or suspended.

Preferably, during said step of exposing said carrier-core shell capsules to said solubilizing medium, said carrier material diffuses through said shell to the outside of said capsules. Preferably, the liquid or solubilizing medium may diffuse through said shell into the capsules. If an active agent was added to the medium, said active agent preferably diffuses into the capsules together with said liquid medium. In particular, said medium may comprise, consist essentially of or consist of said active agent. For example, said liquid medium may be an essence, an essential oil, an extract, for example a plant extract, or any other type of liquid active agent.

Preferably, said carrier core-shell capsules are exposed to said solubilizing medium for a time that is sufficient to allow for diffusion or osmosis of a substantial part of said carrier material through said shell to the outside of said shell.

Preferably, said carrier core-shell capsules are exposed to said liquid and/or solubilizing medium for a duration sufficient to at least partially replace said carrier core of said core-shell capsules by said liquid medium, thereby obtaining core-shell capsules with a liquid core comprising said liquid medium. During this step, a substantial part of said carrier material moves by diffusion and/or osmosis through said shell to the outside of said shell. For example, more than 40 wt.%, preferably more than 50 wt.%, more preferably more than 60 wt.%, most preferably 70 wt.% or more of said carrier material moves out of said carrier-core shell capsules. While it is preferred that essentially all of the carrier material (up to 100 wt.%) is removed from the inside of the capsules, it is generally observed that small or residual amounts of the carrier material remain within the capsules.

The solubilizing medium, optionally comprising an active agent, may move, for example diffuse to the inside of the shell at the same time while said carrier material is moving from the inside to the outside of said shell. In this event, a diffusional exchange occurs through the membrane of said shell of said carrier core-shell capsules. In other words, at least a partial replacement of the carrier material by said solubilizing medium and/or said active agent takes place.

In accordance with the above said, in an aspect or an embodiment, the methods of the invention may be directed to methods of encapsulation and/or methods of providing core-shell capsules having an active ingredient by counter-current flow diffusion.

Alternatively, it is envisaged that the carrier material moves and/or diffuses outside in a first step, and said solubilizing medium and/or an active agent moves and/or diffuses to the inside of the shell in a subsequent, separate step. It is also envisaged that a separate step is provided specifically for letting the active agent, or a solution or dispersion containing it, into the shell, following a preceding step in which the carrier material was removed from the carrier core-shell capsules.

Furthermore, the invention also encompasses the embodiment that the active agent is present together with said carrier core and that during exposure to the solubilizing medium only or substantially only the carrier material diffuses out of the capsule shell, while the active agent is retained within the shell.

By exposure of the carrier-core shell capsules to the solubilizing medium, and/or to a medium comprising an active agent, core-shell capsules comprising a liquid core and/or an active agent are preferably obtained.

In an embodiment, the methods of the invention comprise a step of removing said core-shell capsules with a liquid core, for example as obtained in the preceding step, from said solubilizing and/or liquid medium. Conveniently, said capsules may be separated by filtration, for example.

According to an embodiment, the methods of the invention comprise a step of removing residual liquid and/or solubilizing medium and active agent, if applicable, from the surface of said core-shell capsules.

It is also possible to dry the surface of the core-shell capsules as obtained following the exposure to the medium and/or following the above, optional washing step. Preferably, said core-shell capsules are dried with air or a gas having an appropriate temperature, for example in an air and/or gas stream. Alternatively, or in addition, residual medium or solvent may be removed from the surface of said core-shall capsules with the aid of an appropriate absorbing medium, such silicate powder, for example.

While the capsules obtained above will be useful in many applications, the invention encompasses ways of further processing said capsules, in particular in order to adjust or modify the release characteristics and to control the release.

According to an embodiment, the methods of the invention may comprise the step of chemically and/or physically treating or processing said shell of said core-shell capsules comprising an active agent so as to modify the diffusion properties through the shell of said core shell capsules. If the method of the invention comprises a previous, first step of chemical and/or physical treatment, the present step is preferably a second step of chemical and/or physical treatment. This time, the capsules comprise an active agent and the carrier core has been substantially removed.

Accordingly, the methods of the invention preferably comprise the step of exposing said core-shell capsules to conditions resulting in the change of the diffusion characteristics of the shell of the capsules obtained in the preceding step, so as to reduce the capacity of the active agent to diffuse through said shell and/or to the outside of the capsules, once at least part of the carrier core has been moved out of said shell.

Preferably, the treatment and/or processing of this step results in a reduction of permeability of the membrane of the shell. Preferably, this treatment results in a better retention of the core comprising the active agent within the shell.

Chemical and/or physical treatment for modifying permeability and/or diffusion characteristics of the shell include, for example, exposure to a change of temperatures (exposure to a higher and/or a lower temperature), exposure to pressure, exposure to a pH change, and or applying a coating onto the core-shell capsules.

The methods of the invention may comprise the step of applying a coating on said core-shell capsules comprising an active agent. This step is thus preferably conducted following the step of exposing the cells to said liquid and/or solubilizing medium, optionally after an intermediate washing step. At this stage, the core shell capsules already comprise, in their core, the active agent and the carrier core has been substantially removed.

In case the step of encapsulating said carrier beads involved a coating step, the step of coating said present core-shell capsules comprising the active agent may be referred to as a second coating, while the former coating may be referred to as a first coating.

As the skilled person will understand, if a first and a second coating were applied, said capsules will have a multi-layer shell. The shell of the capsules of the invention may comprise only one (no second coating), two, three or even more layers.

Said chemical and/or physical treatment, for example said coating, results in a change of the pore and/or permeability characteristics and in particular improves the retention of the active agent inside the capsules.

Preferably, if a (second) coating is performed, such coating may be made by way of any suitable film-forming and/or polymeric material. Preferably, a plasticizer is used. For example, said capsules are coated with a substance comprising, consisting essentially of or consisting of a lipid, for example a wax and/or a fat, a lacquer, and/or any other film-forming and/or polymeric material, such as cellulose acetate, cellulose acetate phthalate, for example.

Examples of waxes are carnauba wax, bees wax, castor wax, candellila wax, paraffin wax. examples of other lipids are hydrogenated oil, such as hydrogenated soybean or palm oil, mono glycerides, diglycerides, acetic acid esters, datem, ascorbyl palmitate, calcium stearate, magnesium stearate, and potassium stearate, for example.

The material of the second coating, second or subsequent layer, for example, may be chosen in dependence of the desired release characteristics. The stability, integrity and/or susceptibility of the second or subsequent coating/layer determine the retention and/or the release of the active agent from the core, and may be selected accordingly. If release is to take place only after mechanical damage of the capsule, the material for the second or subsequent coating/layer is preferably selected so as to be relatively stable relatively with respect to fluctuations of environmental parameters, such as the temperature, pH, for example. In addition, in this case the capsule material may be rigid, resisting little or not at all to mechanical constraints and/or pressure.

According to an embodiment, the material of the coating or second coating or layer can be selected in dependence of its melting temperature. In this manner, the release of the core from the capsules can be adjusted or controlled so as to be dependent of the temperature in the environment of the capsules. The capsule may be prepared so as to release its content from the core by change of temperature of the environment, when the wax coating melts away the shell structure becomes permeable again, for example.

Alternatively, or in addition, when a change in the chemical environment occurs, such as a pH change, this may result in a weakening of the shell integrity in dependence of the material that was selected for said (second) coating. An example is an encapsulated surfactant coated with cellulose acetate, for example as a second coating, exposed to an acid environment. Under such an environment, the second layer will be destroyed and the content of the capsule released.

Said first and second coatings or layers of the core shell capsules of the invention may be made from the same or different layers.

The invention relates to capsules that are obtainable by any one of the methods of the invention.

According to an embodiment, the invention provides a core-shell capsule comprising a core comprising an active agent, wherein at least part of said core and/or said active agent has properties of or comprises a liquid and/or at least part of said core and/or said active agent, respectively is a liquid at 60°C, preferably at 50°C, more preferably at 40°C, even more preferably at 35°C, most preferably at 30°C, for example at room temperatures (25°C), or at even lower temperatures, preferably 22°C, 20°C, 15°C, 15°C, 10°C and 5°C.

According to a preferred embodiment, at least a part of said core and/or said active agent has the properties of a liquid or comprises a liquid. Preferably, substances that comprise a liquid encompass compositions that comprise different substances which are present in different conditions of aggregation, respectively, at least one substance being present in the form of a liquid. For example, a gel is considered to be a liquid that is mixed with a solid.

Examples of substances of matter that comprise a liquid and that may function as an active agent and/or core of the capsules of the invention are a gel, a wet spongy structure, a suspension, an emulsion, a dispersion, a colloid, an aerosol, and a foam.

Substances that have properties of a liquid, including liquids, are substances that do not resist strongly to a change of form, but which generally resist to exposure of pressure. Liquids can preferably hardly be compressed. According to a general definition, a liquid is a form of matter with a definite volume but no fixed shape. Examples of substances of matters that are not strictly liquids but which have properties of a liquid and which may be used as at least part of the core and/or active agent in the capsules of the invention may be selected from semi-solids, such as liquid crystals and plastic crystals. Semi-solids, or quasi-solids are also known as amorphous liquids.

According to an embodiment, a liquid, and a substance having the properties of a liquid (such as semi-solids) is a substance having a viscosity in the range of 0.005 to 15'000 centipoises at 60°C, preferably at 50°C, more preferably at 40°C, even more preferably at 35°C, most preferably at 30°C, for example at room temperatures (25°C), or at even lower temperatures, preferably 22°C, 20°C, 15°C, 15°C, 10°C and 5°C.

According to an embodiment, a liquid, and a substance having the properties of a liquid (such as semi-solids) is a substance having a viscosity in the range of 0.005 to 3'000 centipoises at 60°C, preferably at 50°C, more preferably at 40°C, even more preferably at 35°C, most preferably at 30°C, for example at room temperatures (25°C), or at even lower temperatures, preferably 22°C, 20°C, 15°C, 15°C, 10°C and 5°C.

According to an embodiment, a liquid, and a substance having the properties of a liquid (such as semi-solids) is a substance having a viscosity in the range of 0.005 to 1'000 centipoises at 60°C, preferably at 50°C, more preferably at 40°C, even more preferably at 35°C, most preferably at 30°C, for example at room temperatures (25°C), or at even lower temperatures, preferably 22°C, 20°C, 15°C, 15°C, 10°C and 5°C.

According to an embodiment, a liquid, and a substance having the properties of a liquid (such as semi-solids) is a substance having a viscosity in the range of 0.005 to 500 centipoises at 60°C, preferably at 50°C, more preferably at 40°C, even more preferably at 35°C, most preferably at 30°C, for example at room temperatures (25°C), or at even lower temperatures, preferably 22°C, 20°C, 15°C, 15°C, 10°C and 5°C.

According to an embodiment, a liquid, and a substance having the properties of a liquid (such as semi-solids) is a substance having a viscosity in the range of 0.005 to 200 centipoises at 60°C, preferably at 50°C, more preferably at 40°C, even more preferably at 35°C, most preferably at 30°C, for example at room temperatures (25°C), or at even lower temperatures, preferably 22°C, 20°C, 15°C, 15°C, 10°C and 5°C.

Viscosity is preferably determined using a Brookfield DV-II+ viscometer on 0.50 mL of sample at a temperature as specified above, (e.g. 60°C, 50°C, 40°C, 35°C, 30°C, 22°C, 20°C, 15°C, 15°C, 10°C, 5°C, preferably 25°C, as applicable).

According to an embodiment, in the above viscosity ranges expressed in centipoises, the value of 0.005 is replaced by 0.899 centipoises, which is the viscosity of water.

Preferably, at least part of said core, more preferably at least 50wt.%, more preferably at least 60wt.% and most preferably at least 70wt.%, 80wt.% and 90wt.% of said core remains liquid and/or keeps the liquid properties during a shelf life of three months when stored at 25°C and at a relative humidity of 50%.

Preferably, at least part of said core, more preferably at least 50wt.%, more preferably at least 60wt.% and most preferably at least 70wt.%, 80wt.% and 90wt.% of said core remains liquid and/or keeps the liquid properties during a shelf life of six months when stored at 25°C and at a relative humidity of 50%.

Preferably, at least part of said core, more preferably at least 50wt.%, more preferably at least 60wt.% and most preferably at least 70wt.%, 80wt.% and 90wt.% of said core remains liquid and/or keeps the liquid properties during a shelf life of nine months when stored at 25°C and at a relative humidity of 50%.

According to an embodiment, the core of said core-shell capsules comprises residual and detectable amounts of said carrier material. For example, if said carrier material is PEG, said core comprises residual PEG, in particular besides said active agent.

According to an embodiment, the shell of said core-shell capsules comprises cellulose acetate and/or a derivative thereof, such as cellulose acetate phthalate, for example. Other shell materials are mentioned elsewhere in this specification.

According to an embodiment, the shell of said core-shell capsules is multi-layered, comprising at least two, at least three or more distinguishable layers. Said different layers may comprise and/or consist of different materials and/or different compositions. For example, said shell may comprise one or more coatings.

According to an embodiment, the core-shell capsules of the invention have a mean diameter of 1 µm to 1 cm, preferably 200 µm to 9.5 mm, more preferably 400 µm to 9 mm, even more preferably 600 µm to 9 mm, and most preferably 800 µm to 8.5 mm. According to a preferred embodiment, said core-shell capsules have a mean diameter of 1 to 9 mm, 1.5 to 8.5 mm preferably 2 to 8 mm, more preferably 2.5 to 7.5 mm, most preferably 3 to 7 mm, and finally 3.5 to 6.5 mm.

Particles having an average size (diameter) of 1µm up to 1 mm are generally referred to as microcapsules. The invention thus relates to and encompasses microcapsules and methods of their preparation. In addition, the invention also relates to capsules that have an average diameter of more than 1 mm, in particular up to 3 cm, preferably up to 1 cm. One of the advantages of the invention lies in the fact that capsules having a small diameter and capsules having a relatively large diameter can be produced. The present, technology can be used to produce a wide range of capsules sizes in accordance with the requirements and/or preferences for a consumer end product.

Average capsule size may be determined by laser diffraction analysis, in particular as specified in the international standard ISO 13320-1. Preferably, a Malver mastersizer 3000 is used. This methodology is useful for determining average capsules sizes in the range of 0.2 µm to 3.5 mm.

For larger diameters (>3.5 mm), average diameter can be determined by manually measuring, if applicable using a microscope, a statistically representative sample of the capsules. The average is the arithmetic mean for the purpose of this specification.

The micro- and larger capsules of the present invention can be used in a huge variety of applications, taking into account their possibility of selecting a desired size. For example, the invention encompasses consumer end products selected from detergents, food products, clothes, shoes, furniture, vehicles, cars, electronic devices, just to mention a few.

The present invention will now be illustrated by way of examples. These examples do not limit the scope of this invention, which is defined by the appended claims.

### Examples:

### Example 1: Preparation of a support and active agent core

Polyethylene glycol (PEG) 2000 having a melting point of 45-50°C (Merck, Germany) was melted and maintained at a temperature of 60-65°C. 15 wt.% of menthol oil is mixed with the PEG.

A cooling bath was prepared with sunflower oil and kept at 10-15°C. The PEG-menthol mixture was extruded through a nozzle of 1400 µm (Nisco, Zurich, Switzerland) and dropped in a drop-wise manner in the cooling bath.

Beads with a diameter of 2-4 mm were harvested with a sieve from the cooling bath and washed with acetone.

### Example 2: Formation of a shell

15 g cellulose acetate (Sigma Aldrich, CAS no. 9004-35-7) was dissolved in 100 ml acetic acid. Once the cellulose acetate was completely dissolved, 80ml isopropanol was added progressively to the solution. The beads with the core structure of Example 1 were coated in a fluidized bed (MP-1, Aeromatic, Niro, Germany) by atomization of the cellulose acetate solution, with a weight ratio of 90% beads to 10% cellulose acetate.

### Example 3: Replacement of carrier substrate from core

The coated beads of Example 2 were put in a water bath of 70°C and kept therein until liquefaction of the core and absorption of water in the core.

### Example 4: Wax-sealing of the coated beads with liquid core

A part of the beads comprising a shell and a liquid core of Example 3 were coated with castor wax in a fluidized bed (same apparatus as in Example 2), up to a weight ratio of 5% wax and 95% beads.

### Example 5: Cellulose acetate-sealing of the coated beads with liquid core

The remaining part of the beads of Example 3 were fluidized-bed coated with cellulose acetate instead of wax. In particular, 15 g cellulose acetate is dissolved in 100 ml acetic acid. Once the cellulose acetate is completely dissolved, 80ml isopropanol was added progressively to the solution.

The beads obtained in Examples 4 and 5 were stored for 6 months at 25°C at a relative humidity (rH) of 70. Upon crashing individual beads, the liquid content of the beads was released. The active agent of the beads remained liquid in the beads during the 6 months.

### Example 6: Preparation of a support and active agent core

Polyethylene glycol mixture of (PEG) 3000 (melting point of 50-56°) and PEG 1500 (melting point 42-48) in 80/20 proportion was melted and maintained at a temperature of 65-70°C. 15 wt.% of menthol oil was mixed with the PEG mixture. Beads with a diameter of 2-4 mm were prepared and washed as disclosed in Example 1.

### Example 7: Formation of a shell

15 g cellulose phthalate (cellulose acetate phthalate, Sigma Aldrich, CAS no. 9004-38-0) was dissolved in 100 ml acetic acid. Once the cellulose acetate was completely dissolved, 80ml isopropanol was added progressively to the solution. The beads with the core structure of Example 6 were coated in a fluidized bed by atomization of the cellulose phthalate solution, with a weight ratio of 90% beads to 10% cellulose phthalate.

### Example 8: Replacement of carrier substrate from core

The coated beads of Example 7 were put in a water bath of 70°C and kept therein until liquefaction of the core and absorption of water in the core.

### Example 9: Wax-sealing of the coated beads with liquid core

A part of the beads comprising a shell and a liquid core of Example 3 were coated with a mixture of waxes in a fluidized bed, up to a weight ratio of 5% wax and 95% beads. The wax mixture was composed of 33.3% bees wax, 33.3% castor wax and 33.3% carnauba wax, which waxes were melted in a mixture and sprayed on the beads.

### Example 10: Cellulose phthalate-sealing of the coated beads with liquid core

The remaining part of the beads of Example 8 were fluidized-bed coated with cellulose phthalate instead of wax. 15 g cellulose phthalate was dissolved in 100 ml acetic acid. Once the cellulose phthalate was completely dissolved, 80 ml isopropanol was added progressively to the solution.

The beads obtained in Examples 9 and 10 were stored for 6 months. Upon crashing individual beads, the liquid content of the beads was released. The active agent of the beads remained dispersed in the beads during the 6 months.

### Example 11: Preparation of a support and active agent core

PEG 2000 (melting point of 45-50°C) is melted and maintained at a temperature of 60-65°C. 15 wt.% of encapsulated tea-tree oil in chitosan in solid form is mixed with the PEG.

A cooling bath is prepared with sunflower oil and kept at 10-15°C. The PEG-tea-tree encaspulated mixture are extruded through a nozzle of 500 µm and dropped in a drop-wise manner into the cooling bath.

Beads with a diameter of 1-2 mm are harvested with a sieve from the cooling bath and washed with acetone.

### Example 12: Formation of a shell

The beads with the core structure of Example 11 are coated with a weight ratio of 90% beads to 10% cellulose acetate exactly as disclosed in Example 2.

### Example 13: Replacement of carrier substrate from core

The coated beads of Example 12 are put in a water bath of 70°C and kept therein until liquefaction of the core and absorption of water into the core.

### Example 14: Fat-sealing of the coated beads with liquid core

A part of the beads comprising a shell and a liquid core of Example 13 are coated with fat in a fluidized bed, up to a weight ratio of 5% fat and 95% beads. The fat is Bergazid fat (C1852, from Berg and Schmidt, Hamburg, Germany), which is melted and sprayed on the beads.

### Example 15: Cellulose acetate-sealing of the coated beads with liquid core

The remaining part of the beads of Example 13 are fluidized-bed coated with cellulose acetate as disclosed in Example 5.

The beads obtained in Examples 14 and 55 are stored for 6 month at 25 ° C at 70 % rH. Upon crashing individual beads, the liquid content of the beads is released. The active agent of the beads remains liquid in the beads for up to 6 months.

### Example 16: Preparation of a support and active agent core

Polyethylene glycol mixture of (PEG) 3000 (melting point of 50-56°) and PEG 1500 (melting point 42-48) in 80/20 proportion is melted and maintained at a temperature of 65-70°C. No active ingredient is added at this stage. Beads with a diameter of 2-4 mm are prepared and washed as disclosed in Example 1.

### Example 17: Formation of a shell

The beads with the core structure of Example 16 are coated in a fluidized bed with cellulose phthalate as disclosed in Example 7.

### Example 18: Replacement of carrier substrate from core

The coated beads of Example 17 are put in a emulsion bath of 60°C composed of water 90% w/w, menthol oil 8%w/w, tween20 1.5% w/w, span 40 0.5% w/w and kept therein until liquefaction of the core and absorption of the emulsion in the core.

### Example 19: Wax-sealing of the coated beads with liquid core

A part of the beads comprising a shell and a liquid core of Example 18 are coated with a wax mixture as used in Example 9 in a fluidized bed, up to a weight ratio of 5% wax and 95% beads.

### Example 20: Cellulose phthalate-sealing of the coated beads with liquid core

The remaining part of the beads of Example 18 are fluidized-bed coated with cellulose phthalate as disclosed in Example 10.

The beads obtained in Examples 19 and 20 are stored for 12 month at 70°HR, at room temperature. Upon crashing individual beads, the liquid content of the beads is released. The active agent of the beads remained dispersed in the beads during the storage time.

### Example 21: Preparation of a support and active agent core

Beads with a diameter of 2-4 mm were prepared and washed as disclosed in Example 16.

### Example 22: Formation of a shell

The beads with the core structure of Example 21 were fluidized bed coated with cellulose phthalate solution as disclosed in Example 7.

### Example 23: Replacement of carrier substrate from core

The coated beads of Example 22 were put in a flavor bath of 60°C composed of thyme essential oil of and kept therein until liquefaction of the core and absorption of the flavor in the core.

### Example 24: Wax-sealing of the coated beads with liquid core

A part of the beads comprising a shell and a liquid core of Example 3 were coated with the wax mixture disclosed in Example 9 in a fluidized bed.

### Example 25: Cellulose phthalate-sealing of the coated beads with liquid core

The remaining part of the beads of Example 23 were fluidized-bed coated with cellulose phthalate as disclosed in Example 10.

The beads obtained in Examples 24 and 25 were stored for 12 month at 70°HR, at room temperature. Upon crashing individual beads, the liquid content of the beads was released. The active agent of the beads remained dispersed in the beads for up to 12 months.

## Claims

1. A method of encapsulation, the method comprising the steps of:
- preparing carrier beads from a carrier material,
- encapsulating said carrier beads with an appropriate film forming and/or polymeric material so as to obtain carrier core-shell capsules;
- exposing said carrier core-shell capsules to a liquid and/or solubilizing medium, wherein said carrier material is soluble or dispersible in said medium;
- removing said capsules obtained in the preceding step from said medium, so as to obtain core-shell capsules comprising an active agent;
wherein said medium and/or said carrier material comprises an active agent.

2. The method of claim 1, wherein the shell of said carrier core-shell capsules has or is further processed to have membrane characteristics allowing for diffusion and/or osmosis of said medium and/or of a liquefied, dissolved and/or dispersed carrier material through said shell.

3. The method of any one of the preceding claims, wherein said film forming and/or polymeric material forming said shell of said carrier-core shell capsules is a material comprising pores and/or wherein said shell is semi-permeable.

4. The method of any one of the preceding claims, wherein said film-forming and/or polymeric material is or comprises cellulose acetate and/or a derivative thereof, such as cellulose acetate phthalate.

5. The method of any one of the preceding claims, further comprising the step of chemically or physically treating or processing said shell of said core-shell capsules comprising an active agent so as to modify the diffusion properties through the shell of said core shell capsules.

6. The method of any one of the preceding claims, comprising the step of applying a coating on said core-shell capsules.

7. The method of any one of the preceding claims, wherein during said step of exposing said carrier-core shell capsules to said liquid medium, said carrier material diffuses through said shell to the outside of said capsules, and the liquid medium diffuses through said shell into the capsules.

8. The method of any one of the preceding claims, wherein said carrier material of said carrier beads is selected from PEG, water soluble saccharides, in particular water soluble mono-, di- , oligo- and polysaccharides.

9. The method of any one of the preceding claims, wherein said carrier material of said carrier beads, said active agent and said liquid medium are selected so that said carrier material and said active agent are both soluble and/or dispersible in said liquid medium, optionally following chemical and/or physical treatment of said liquid medium.

10. The method of any one of the preceding claims, wherein at least a part of the core of said core-shell capsules comprising an active agent and/or said active agent has properties of a liquid and/or at least part of said core and/or said active agent, respectively is a liquid, preferably at a temperature of 35°C, preferably at 25°C.

11. Capsules obtainable by the method of any one of claims 1 to 10.

12. A capsule comprising a core at least part of which is liquid, comprises a liquid or has properties of a liquid at 35°C, preferably at room temperature (25°C), and a shell, and comprising an active agent, said shell comprising cellulose acetate and/or a derivative thereof.

13. The capsule of claim 12, wherein said shell is coated.

14. The capsules of claim 12 and 13, wherein said core further comprises residual PEG.

15. The capsules of any one of claims 1 to claim 14, having a mean diameter of 1 µm to 1 cm.
